# EUROPEAN PATENT APPLICATION

(11) **EP 4 202 064 A1**
(43) Date of publication of application: **28.06.2023**
(21) Application number: 21857715.3
(22) Date of filing: 18.08.2021
(51) Int. Cl.: C12Q 1/70, C12Q 1/6844

(54) **KIT AND METHOD FOR ISOTHERMAL RAPID DETECTION OF SARS-COV-2 VIRUS NUCLEIC ACID**

(30) Priority: 18.08.2020 CN 202010833690
(71) Applicant: Shanghai Jiao Tong University, Shanghai 200240 (CN)
(72) Inventor: FENG, Yan, Shanghai 200240 (CN); LI, Zhonglei, Shanghai 200240 (CN); YE, Xingyu, Shanghai 200240 (CN); GUO, Xiang, Shanghai 200240 (CN); HUANG, Jun, Shanghai 200240 (CN); LIU, Tao, Shanghai 200240 (CN)
(74) Representative: Viering, Jentschura & Partner mbB Patent- und Rechtsanwälte
(86) International application number: PCT/CN2021/113348
(87) International publication number: WO 2022/037623

(57) **Abstract**

The present invention provides a kit and method for isothermal rapid detection of a SARS-CoV-2 virus nucleic acid. The detection method comprises: (a) providing a sample to be detected containing a target nucleic acid molecule; (b) mixing said sample with a cleavage reagent or a cleavage buffer solution containing the cleavage reagent so as to form a detection system, wherein the cleavage reagent comprises two guide ssDNAs, a gene editing enzyme (Ago), and a first reporter nucleic acid molecule, the first reporter nucleic acid molecule has a fluorescent group and a quenching group, and the two guide ssDNAs are adjacent to each other; and (c) performing fluorescence detection on the detection system so as to obtain a fluorescence signal value, and if the fluorescence signal value is detected in the detection system, indicating that the target nucleic acid molecule exists in said sample, and if the fluorescence signal value is not detected in the detection system, indicating that the target nucleic acid molecule does not exist in said sample.

## Description

### Technical field

The present invention belongs to the field of biotechnology, specifically, the invention relates to the SARS-CoV-2 virus nucleic acid isothermal rapid detection kit and detection method.

### Background

The outbreak of the novel coronavirus (2019-nCoV, hereinafter referred to as "new coronavirus") has spread throughout the country and the WHO has declared the 2019-nCoV outbreak as a public health emergency of international concern. The Chinese government has taken active action and launched an emergency prevention and control program. The current outbreak requires high-technology input from the diagnosis to the treatment of infected patients. Currently, the accurate and quantitative detection of new coronaviruses, especially low abundance viruses, has a positive impact on patient diagnosis, subsequent control and treatment. Since the launch of viral genome sequencing results, the development of detect products for specific nucleic acid sequences has become a focus of attention. To date, seven in vitro diagnostic companies have launched their products on the market, and more than twenty others have also launched detect products. The real-time fluorescent PCR (reverse transcription PCR) method is a clinical standard for the detection of COVID-19 virus nucleic acid. The principle of reverse transcription PCR (i.e., reverse transcription-polymerase chain reaction) is to extract total RNA from tissues or cells, use the mRNA as a template, Oligo(dT) or random primers are used to reverse transcribe into cDNA using reverse transcriptase, and then use the cDNA as a template for PCR amplification to obtain the target gene or detect gene expression. In terms of clinical application, nucleic acid diagnostic kits have cost and time advantages over conventional genomic sequencing analysis, but as they are mostly based on fluorescent PCR methods, there are still problems of long testing time (around 2 hours), high price (200 yuan/time), expensive equipment (300,000-600,000 yuan for Q-PCR instruments), and high professional requirements for operators, therefore, at present, the current diagnosis of confirmed and suspected patients remains a limiting factor for subsequent prevention, control and treatment. The development of new diagnostic techniques for new infectious diseases, and the development of POCT (instant field testing) instruments to accompany them, to achieve rapid (less than 1 hour), low-cost and sensitive products that can also be applied at the grassroots level, are urgent scientific challenges.

Principle of the one-step reverse transcription LAMP nucleic acid amplification technique: Reverse Transcription LAMP, the combination of Reverse Transcription and Loop-Mediated Isothermal Amplification, specifically designed for the rapid amplification of RNA. LAMP is a novel isothermal nucleic acid amplification method that has only been available since 2000. It utilizes 4 or 6 template-specific primers and a DNA polymerase with strand-displacement capability to complete amplification in 15-60 minutes at isothermal conditions (around 63°C). The technique is comparable or even superior to reverse transcription PCR in terms of sensitivity, specificity and detection range, and does not require thermal denaturation of the template, temperature cycling, electrophoresis and UV observation, and does not rely on any specialized instrumentation. It has been successfully applied to the rapid detection of human, animal and plant, bacterial, viral, parasitic, fungal and other pathogens. Recently, the Reverse Transcription LAMP method (Reverse Transcription Loop Mediated Isothermal Amplification) has also been applied to the detection of SARS-CoV-2 virus nucleic acid. However, the reverse transcription LAMP method has problems in detecting novel coronaviruses, such as high false positives and poor reliability.

There are still many limitations of CRISPR-based nucleic acid detection technology, such as the limitation of CRISPR to detect gene sequences, the difficulty of multiplex detection, the relatively complex design and expensive synthesis of crRNA, and the easy degradation of crRNA, which are still the limitations of its market entry.

Therefore, there is an urgent need to develop rapid, simple, efficient, highly sensitive and highly accurate methods for the detection of SARS-CoV-2.

### Summary of the invention

The purpose of the present invention is to provide a fast, simple, efficient, highly sensitive and accurate method for the detection of SARS-CoV-2.

In a first aspect of the present invention, it provides a method for detecting a target nucleic acid molecule, comprising the steps of:
(a) providing a sample to be tested comprising a target nucleic acid molecule, the target nucleic acid molecule comprises a single stranded DNA;
(b) mixing the sample to be tested with a cleavage reagent or a cleavage buffer containing the cleavage reagent, thereby forming a detection system, wherein the cleavage reagent comprises: 2 guide ssDNA, a gene editing enzyme (Ago), and a first reporter nucleic acid molecule, the first reporter nucleic acid molecule bearing a fluorescent group and a quencher, and wherein the 2 guide ssDNA are adjacent to each other; and
(c) performing a fluorescence detection on the detection system, thereby obtaining a fluorescence signal value, wherein the detection of a fluorescence signal value in the detection system indicates the presence of a target nucleic acid molecule in the sample, and the absence of a fluorescence signal value in the detection system indicates the absence of a target nucleic acid molecule in the sample.

In another preferred embodiment, the sample to be tested comprises an unamplified sample as well as an amplified (or nucleic acid amplified) sample.

In a further preferred embodiment, the sample to be tested is a sample obtained by amplification.

In a further preferred embodiment, the amplification is selected from the group consisting of: PCR amplification, LAMP amplification, RPA amplification, ligase chain reaction, branched DNA amplification, NASBA, SDA, transcription-mediated amplification, rolling loop amplification, HDA, SPIA, NEAR, TMA, SMAP2, EXPAR, and a combination thereof.

In another preferred example, the first nucleotide at the 5' end of the respective guide ssDNA is T.

In another preferred embodiment, the lengths of the guide ssDNA are each independently 10-60 nt, preferably 10-40 nt, more preferably, 13-20 nt.

In another preferred embodiment, the guide ssDNA may be the same or different.

In another preferred embodiment, the guide ssDNA may have the same or different lengths.

In a further preferred example, the guide ssDNA is a phosphorylated single stranded DNA molecule.

In a further preferred example, the guide ssDNA is a 5'-phosphorylated single-stranded DNA molecule; and/or a 3'-phosphorylated single-stranded DNA molecule.

In another preferred example, the gene editing enzyme Ago is selected from the group consisting of: *PfAgo (Pyrococcus furiosus Ago), MfAgo (Methanocaldococcus fervens Ago), TcAgo (Thermogladius calderae Ago), TfAgo ( Thermus filiformis Ago), AaAgo (Aquifex aeolicus Ago), TpAgo (Thermus parvatiensis Ago),* and a combination thereof.

In a further preferred example, the gene editing enzyme Ago comprises *PfAgo (Pyrococcus furiosus Ago).*

In a further preferred embodiment, the Ago comprises wild type and mutant Ago.

In a further preferred embodiment, the gene editing enzyme has an operating temperature of 87-99°C.

In another preferred embodiment, the target nucleic acid molecule (or amplification product thereof) is cleaved by the gene editing enzyme Ago to produce a secondary guide ssDNA.

In another preferred embodiment, the secondary guide ssDNA has a length of 10-60 nt, preferably 10-40 nt, more preferably, 15-17 nt.

In a further preferred example, the secondary guide ssDNA is complementary to the sequence of the fluorescent reporter nucleic acid (a first reporter nucleic acid molecule).

In another preferred embodiment, after complementary binding of the secondary guide ssDNA to the sequence of the fluorescent reporter nucleic acid (a first reporter nucleic acid molecule), directing the gene editing enzyme Ago to cleave the fluorescent reporter nucleic acid (a first reporter nucleic acid molecule), thereby generating a detectable signal (e.g. fluorescence).

In a further preferred embodiment, the fluorescent group and quencher are each independently located at the 5' end, 3' end of the fluorescent reporter nucleic acid.

In another preferred embodiment, the fluorescent group is selected from the group consisting of: FAM, HEX, CY3, CY5, ROX, VIC, JOE, TET, Texas Red, NED, TAMRA, and a combination thereof.

In a further preferred example, the quencher is selected from the group consisting of: TAMRA, BHQ, DABSYL, and a combination thereof.

In another preferred embodiment, the fluorescent reporter nucleic acid (a first reporter nucleic acid molecule) has a length of 9-100nt, preferably 10-60nt, more preferably 15-40nt.

In another preferred embodiment, the target nucleic acid molecule is selected from the group consisting of: a nucleic acid molecule of a pathogenic microorganism, a nucleic acid molecule with a genetic mutation, and a specific target nucleic acid molecule.

In a further preferred embodiment, the pathogenic microorganism comprises a virus, a bacterium, a chlamydia, a mycoplasma.

In a further preferred embodiment, the virus comprises a plant virus or an animal virus.

In a further preferred embodiment, the virus comprises: coronavirus, influenza virus, HIV, hepatitis virus, parainfluenza virus.

In a further preferred embodiment, the virus is a coronavirus.

In a further preferred example, the virus is selected from the group consisting of SARS, SARS-CoV-2 (COVID-19), HCoV-229E, HCoV-OC43, HCoV-NL63, HCoV-HKU1, Mers-CoV, and a combination thereof.

In a further preferred example, the target nucleic acid molecule comprises wild-type or mutant DNA.

In a further preferred embodiment, the target nucleic acid molecule comprises a single-stranded cDNA.

In a further preferred embodiment, the target nucleic acid molecule comprises DNA obtained by reverse transcription or amplification of RNA, such as cDNA.

In a further preferred embodiment, the target nucleic acid molecule comprises a single base mutation present in a SARS-CoV-2 virus.

In a further preferred embodiment, the target nucleic acid molecule comprises a Single Nucleotide Polymorphism (SNP) mutant of the ORF1ab, N, E, S genes etc. of the SARS-CoV-2 virus.

In another preferred embodiment, the target nucleic acid molecule comprises a mutation at position 614 of the spike protein sequence expressed by the S gene of the SARS-CoV-2 virus with an amino acid mutation type D614G, i.e. the amino acid at position 614 is changed from aspartate (D) to glycine (G).

In another preferred embodiment, the target nucleic acid molecule further comprises, at the nucleotide level, a mutation of nucleotide at position 23,403 from A to G in the SARS-CoV-2 virus.

In a further preferred embodiment, the target nucleic acid molecule further comprises, at the nucleotide level, a mutant of T8782C, G11083T, G26144T, C28144T of the SARS-CoV-2 virus.

In a further preferred embodiment, the target nucleic acid molecule further comprises, at the amino acid level, the mutant N501Y, E484Q, L452R of the SARS-CoV-2 virus.

In a further preferred embodiment, the method is used to detect whether the nucleic acid at the target site is at a SNP, point mutation, deletion, and/or insertion.

In a further preferred embodiment, the method is used for typing the virus, i.e. determining whether the virus is wild type or mutant.

In a further preferred embodiment, the cleavage reagent further comprises 2 additional reporter nucleic acid molecules (a second reporter nucleic acid molecule and a third reporter nucleic acid molecule) when used for typing the virus.

In a further preferred embodiment, the second reporter nucleic acid molecule, the third reporter nucleic acid molecule are each independently 9-200 nt in length, preferably 10-60 nt, more preferably 15-40 nt.

In another preferred embodiment, after the second reporter nucleic acid molecule binds complementarily to the sequence of the secondary guide ssDNA produced by the wild-type virus, directing the gene editing enzyme Ago to cleave the second reporter nucleic acid molecule, thereby producing a detectable signal (e.g. fluorescence).

In another preferred example, the third reporter nucleic acid molecule does not bind complementarily to the sequence of the secondary guide ssDNA produced by the wild-type virus, and therefore the gene editing enzyme Ago does not cleave the third reporter nucleic acid molecule, thereby failing to produce a detectable signal (e.g. fluorescence).

In another preferred example, the second reporter nucleic acid molecule does not bind complementarily to the sequence of the secondary guide ssDNA produced by the mutant virus, and therefore the gene editing enzyme Ago does not cleave the second reporter nucleic acid molecule, thereby failing to produce a detectable signal (e.g. fluorescence).

In another preferred example, after complementary binding of the third reporter nucleic acid molecule to the sequence of the secondary guide ssDNA produced by the mutant virus, directing the gene editing enzyme Ago to cleave the second reporter nucleic acid molecule, thereby generating a detectable signal (e.g. fluorescence).

In another preferred embodiment, the fluorescence detection in step (c) is performed using a Microplate Reader or a fluorescence spectrophotometer.

In a further preferred embodiment, the detection system further contains a buffer or buffering agent.

In another preferred embodiment, the sample to be tested is the sample to be tested obtained by amplification with primers selected from the group below.

In another preferred example, the primer is any one of primer pair selected from the group consisting of:

| | | | |
|---|---|---|---|
| prim er pair 1 | F3 | ATCAGTACTAGTGCCTGTG | SEQ ID NO.1 |
| | B3 | TGTTGTCTGTACTGCCGT | SEQ ID NO.2 |
| | FIP | TAGTTGTGATCAACTCCGCGAATGCACTTACAC CGCAAAC | SEQ ID NO.3 |
| | BIP | AGCACAAGTTGTAGGTATTTGTACATGCCACAT AGATCATCCAAAT | SEQ ID NO.4 |
| | LF1 | CAGCTGATGCACAATCGTTTTTA | SEQ ID NO.5 |
| | LB1 | CCTTTTAAGTCACAAAATCCTTTAG | SEQ ID NO.6 |
| prim er pair 2 | F3 | GAATTTAGCAAAACCAGCTACT | SEQ ID NO.7 |
| | B3 | GTGTTGTCTGTACTGCCG | SEQ ID NO.8 |
| | FIP | CGTTTTTAAACGGGTTTGCGGTATACGACATCA GTACTAGTGC | SEQ ID NO.9 |
| | BIP | TGTAAAACCCACAGGGTCATTAGCCCAAATCCT AAAGGATTTTGTG | SEQ ID NO.10 |
| | LF1 | TGCAGCCCGTCTTACACC | SEQ ID NO. 11 |
| | LB1 | CAAGTTGTAGGTATTTGTACATACT | SEQ ID NO.12 |

| | | | |
|---|---|---|---|
| prim er pair 3 | F3 | 5'-TGTTCTTGCTCGCAAACA-3' | SEQ ID NO.13 |
| | B3 | 5'-GTGTTGTAAATTGCGGACAT-3' | SEQ ID NO.14 |
| | FIP | 5'-ACACATGACCATTTCACTCAATACTAGCTTGTCAC ACCGTTTC-3' | SEQ ID NO.15 |
| | BIP | 5'-CATTTGTCAAGCTGTCACGGCGCAATTTTGTTACCA TCAGTAG-3' | SEQ ID NO.16 |
| | LF2 | 5'-ACACTCATTAGCTAATC-3' | SEQ ID NO.17 |
| | LB2 | 5'-CAATGTTAATGCACTTTT-3' | SEQ ID NO.18 |
| prim er pair 4 | F3 | 5'-CGCAAACATACAACGTGTTG-3' | SEQ ID NO.19 |
| | B3 | 5'-GTGTTGTAAATTGCGGACAT-3' | SEQ ID NO.20 |
| | FIP | 5'-ACACATGACCATTTCACTCAATACTGCTTGTCACA | SEQ ID NO.21 |
| | | CCGTTTCT-3' | |
| | BIP | 5'-CATTTGTCAAGCTGTCACGGCGGCAATTTTGTTACC ATCAGT-3' | SEQ ID NO.22 |
| | LF1 | 5'-CACACTCATTAGCTAATCTAT-3' | SEQ ID NO.23 |
| | LB1 | 5'-CAATGTTAATGCACTTTTATC-3' | SEQ ID NO.24 |

| | | | |
|---|---|---|---|
| prim er pair 5 | F3 | 5'-TCTGCCGAAAGCTTGTGTT-3' | SEQ ID NO.25 |
| | B3 | 5'-CGTAGTCGCAACAGTTCAAG-3' | SEQ ID NO.26 |
| | FIP | 5'-GCCAACAACAACAAGGCCAAACTCAGTACGTTTTTG CCGAGG-3' | SEQ ID NO.27 |
| | BIP | 5'-GCATCACCGCCATTGCCAGCCAACTCCAGGCAGCAG TAG-3' | SEQ ID NO.28 |
| | LF1 | 5'-GCTGCTGAGGCTTCTAAGAAG-3' | SEQ ID NO.29 |
| | LB1 | 5'-ATTCTAGCAGGAGAAGTTCCC-3' | SEQ ID NO.30 |
| prim er pair 6 | F3 | 5'-TCTGCCGAAAGCTTGTGTT-3' | SEQ ID NO.31 |
| | B3 | 5'-CAGTCAAGCCTCTTCTCGT-3' | SEQ ID NO.32 |
| | FIP | 5'-CCAACAACAACAAGGCCAAACTGGCAGTACGTTTTT GCCGAG-3' | SEQ ID NO.33 |
| | BIP | 5'-ACCGCCATTGCCAGCCATTCTAACGTAGTCGCAACA GTTCA-3' | SEQ ID NO.34 |
| | LF1 | 5'-TGCTGCTGAGGCTTCTAAGAA-3' | SEQ ID NO.35 |
| | LB1 | 5'-GTTCCCCTACTGCTGCCTGGA-3' | SEQ ID NO.36 |

| | | | |
|---|---|---|---|
| prim er pair 7 | F3 | TTGATGAGCTGGAGCCA | SEQ ID NO.37 |
| | B3 | CACCCTCAATGCAGAGTC | SEQ ID NO.38 |
| | FIP | GTGTGACCCTGAAGACTCGGTTTTAGCCACTGACTCGGA TC | SEQ ID NO.39 |
| | BIP | CCTCCGTGATATGGCTCTTCGTTTTTTTCTTACATGGCTC TGGTC | SEQ ID NO.40 |
| | LF1 | ATGTGGATGGCTGAGTTGTT | SEQ ID NO.41 |
| | LB1 | CATGCTGAGTACTGGACCTC | SEQ ID NO.42 |
| prim er pair 8 | F3 | GGAGCCAGAGACCGACA | SEQ ID NO.43 |
| | B3 | CTCAGGAAGGCCCACTA | SEQ ID NO.44 |
| | FIP | TTACTTGGGTGTGACCCTGAAGACACGGGAGCCACTGA CT | SEQ ID NO.45 |
| | BIP | AAGAAAAGGCCTGTTCCCTGGAAGGCCACCAAGAGACA AT | SEQ ID NO.46 |
| | LF1 | TGGATGGCTGAGTTGTTGCG | SEQ ID NO.47 |
| | LB1 | GCCCAAAGGACTCTGCATTGA | SEQ ID NO.48 |

In a further preferred example, the guide ssDNA is selected from the group consisting of

| | | |
|---|---|---|
| 2019-nCoV ORF 1a-g**DNA 1** | 5'-**P**-TGTATGTGGAAAGGTT-3' | SEQ ID NO.49 |
| 2019-nCoV ORF 1a-**gDNA 2** | 5'-**P**-TGTCTGTACCGTCTGC-3' | SEQ ID NO.50 |
| 2019-nCoV ORF 1b-**gDNA 1** | 5'-**P**-TTGATGAGGTTCCACC-3' | SEQ ID NO.51 |
| 2019-nCoV ORF 1b-**gDNA 4** | 5'-**P**-TCAGTTGTGGCATCTC-3' | SEQ ID NO.52 |
| 2019-nCoV ORF 1b-**gDNA 2** | 5'-**P**-TAGGTGGAACCTCATC-3' | SEQ ID NO.53 |
| 2019-nCoV ORF 1b-**gDNA 3** | 5'-**P**-TGGAGATGCCACAACT-3' | SEQ ID NO.54 |
| 2019-nCoV **N-gDNA** 1 | 5'-**P**-TCTTGACAGATTGAAC-3' | SEQ ID NO.55 |
| 2019-nCoV **N-gDNA** 2 | 5'-**P**-TCTTGCTTTGCTGCTG-3' | SEQ ID NO.56 |
| RNase **P-gDNA1** | 5'-**P**-TACTCAGCATGCGAAG-3' | SEQ ID NO.57 |
| RNase **P-gDNA2** | 5'-**P**-TGCCATATCACGGAGG-3' | SEQ ID NO.58 |
| 2019-nCoV ORF 1a-Reporter 1 | 5'-FAM-AGTCTGTACCGTCTGCG GTATGTGGAAAGG-BHQ1-3' | SEQ ID NO.59 |
| 2019-nCoV ORF 1b-Reporter 1 | 5'-FAM-CAGTTGTGGCATCTCCT GATGAGGTTCCAC-BHQ1-3' | SEQ ID NO.60 |
| 2019-nCoV ORF 1b-Reporter 2 | 5'-FAM-GTGGAACCTCATCAGG AGATGCCACAACTG-BHQ1-3' | SEQ ID NO.61 |
| 2019-nCoV N-Reporter 1 | 5'-FAM-CTCTTGCTTTGCTGCTG CTTGACAGATTGA-BHQ1-3' | SEQ ID NO.62 |
| RNase P-Reporter1 | 5'-FAM-CTCAGCATGCGAAGAG CCATATCACGGAGG-BHQ1-3' | SEQ ID NO.63 |
| RNase P-Reporter2 | 5'-VIC-CTCAGCATGCGAAGAGC CATATCACGGAGG-BHQ1-3' | SEQ ID NO.64 |

wherein P is the phosphorylation modification at the 5' end of the oligonucleotide.

In another preferred embodiment, the sample to be tested is a nucleic acid sample prepared from a sample selected from the group consisting of: throat swab, broncholveolr lvge fluid, nose swab, urine, faeces, body fluid, and a combination thereof.

In a further preferred embodiment, the method is an in vitro method.

In a further preferred embodiment, the method is non-diagnostic and non-therapeutic.

In a second aspect of the present invention, it provides a kit for the detection of a target nucleic acid molecule, the kit comprising.
(a) an amplification reagent for amplifying a target nucleic acid molecule, the amplification reagent comprising: a primer pair for amplifying a target nucleic acid molecule, the primer pair being used to carry out a specific amplification reaction based on the target nucleic acid molecule, thereby producing a specific nucleic acid amplification product, thereby producing a specific nucleic acid amplification product.
(b) a cleavage reagent or a cleavage buffer comprising the cleavage reagent, wherein the cleavage reagent comprises: 2 guide ssDNA, a gene editing enzyme (Ago), and a first reporter nucleic acid, the first reporter nucleic acid bearing a fluorescent group and a quencher, and wherein the 2 guide ssDNA are adjacent to each other.

In a further preferred example, the kit further comprises a second reporter nucleic acid, a third reporter nucleic acid.

In a further preferred embodiment, the kit further comprises:
(c) a reverse transcription reagent for a reverse transcription reaction, the reverse transcription reagent comprising: a reverse transcriptase or a Bst enzyme (or a mutant thereof) possessing reverse transcriptase activity.

In a further preferred example, the kit comprises the following preferred combination of reagents:
For SARS-CoV-2 ORF1a:
   Primer pair 1: SEQ ID No: 1-6;
   fluorescent reporter nucleic acid: SEQ ID No. 59;
   ssDNA: SEQ ID No: 49 and 50;
For SARS-CoV-2 ORFlb gene:
   Primer pair 3: SEQ ID Nos: 13-18;
   Fluorescent reporter nucleic acid: SEQ ID No. 60;
   ssDNA: SEQ ID No: 51 and 52;
   For SARS-CoV-2 N gene;
   Primer pair 5: SEQ ID Nos: 25-30;
   Fluorescent reporter nucleic acid: SEQ ID No. 62;
   ssDNA: SEQ ID Nos. 55 and 56;
For RNase P reference gene:
   Primer pair 7: SEQ ID No: 37-42;
   Fluorescent reporter nucleic acids: SEQ ID Nos. 63 and 64;
   ssDNA: SEQ ID Nos: 57 and 58.

In a third aspect of the present invention, it provides a kit for detecting a target nucleic acid molecule, the kit comprising:
(i) a first container and a guide ssDNA in the first container, the guide ssDNA being 2 and the 2 guide ssDNA being adjacent to each other;
(ii) a second container and a gene editing enzyme (Ago) in the second container;
(iii) a third container and a first reporter nucleic acid in the third container, the first reporter nucleic acid bearing a fluorescent group and a quencher;
(iv) a fourth container and an amplification reagent for amplifying a target nucleic acid molecule in the fourth container; and
(v) optionally a fifth container and a buffer in the fifth container;
(vi) optionally a PCR tube and a liner pipe corresponding to the PCR;
wherein the target nucleic acid molecule comprises a single stranded DNA.

In another preferred example, said kit further comprises a sixth container, and a second reporter nucleic acid and a third reporter nucleic acid in the sixth container.

In another preferred embodiment, the amplification reagent comprises: a primer pair for amplifying a target nucleic acid molecule, the primer pair being used to perform a specific amplification reaction based on the target nucleic acid molecule, thereby generating a specific nucleic acid amplification product.

In a further preferred embodiment, the buffer in the fifth container comprises: a buffer for the amplification reaction and a buffer for the enzyme to perform the enzymatic digestion.

In another preferred embodiment, the buffer for the amplification reaction comprises: Bst buffer (Tris-Hcl, EDTA, NaCl or KCl) , MgSO₄, dNTP, DNase/RNase free H₂O.

In a further preferred example, the buffer for the enzyme to perform the enzymatic digestion comprises Bst buffer or Reaction buffer (Tris-Hcl, EDTA, NaCl or KCl), MnCl₂, DNase/RNase free H₂O.

In another preferred example, any two, three, four or all of the first container, second container, third container, fourth container, fifth container, sixth container may be the same or different containers.

It should be understood that within the scope of the present invention, the above-mentioned technical features of the present invention and the technical features described in detail below (such as embodiments) can be combined with each other to form a new or preferred technical solution. Due to space constraints, I will not repeat them here.

### Description of Drawings

Figure 1 shows a real picture of a modified liner pipe and PCR tube combination, including a stereoscopic schematic, a top view cross-sectional schematic; 1 is the liner pipe body, 2 is the PCR tube body, 3 is the liner pipe reaction chamber, 4 is the PCR tube reaction chamber, 101 is the liner pipe wall (thickness 3-7 mm), 102 is the liner pipe bottom hole (aperture 3-5 mm), 103 is the gap between the liner pipe wall and the PCR tube (5-10 mm at the largest gap).
Figure 2 shows a schematic diagram of the "one-tube" dual nucleic acid rapid assay for reverse transcription LAMP-RADAR coupling.
Figure 3 shows a schematic diagram of the reverse transcription LAMP-RADAR assay.
Figure 4 shows the sensitivity of the LAMP-RADAR assay for SARS-CoV-2 virus.
Figure 5 shows a schematic diagram of the fluorescent real-time assay for selected SARS-CoV-2 clinical samples.
Figure 6 shows the results of the SARS-CoV-2 dual LAMP-RADAR specificity assay.
Figure 7 shows a schematic diagram of the principle of SARS-CoV-2 LAMP-RADAR typing assay.
Figure 8 shows the SARS-CoV-2 gene sequence D614G mutation site.

### DETAILED DESCRIPTION

Through extensive and intensive research, the present inventors have developed for the first time a nucleic acid detection method for target nucleic acid molecules (e.g. the target nucleic acid molecule of the new coronavirus SARS-CoV-2) that is highly sensitive, easy to perform, low cost of detection, short time consuming and highly accurate. The invention performs a reverse transcription loop-mediated isothermal amplification reaction (reverse transcription LAMP) on the target nucleic acid molecule and then uses the properties of the Ago enzyme, i.e. after the two adjacent primary guide ssDNA (guide ssDNA) with non-spaced sequences mediated cleavage for the first time, the broken 5' nucleic acid fragment can be utilized again by the Ago enzyme at a suitable reaction temperature (e.g. about 90-98 degrees) to cleave the complementary fluorescent reporter nucleic acid strand, thereby determining whether the sample contains the target nucleic acid molecule based on the fluorescence. The results show that the method of the invention allows rapid and highly sensitive and accurate detection of target nucleic acid molecules, thus providing assistance in pathogen detection, genotyping, disease course monitoring, etc. The present invention has been completed on this basis.

### Terms

The term "LAMP" refers to Loop-mediated isothermal amplification, an isothermal nucleic acid amplification technique suitable for genetic diagnostics.

The term "PCR" refers to polymerase chain reaction, a technique suitable for the amplification of target nucleic acids.

As used herein, the term "secondary cleavage" means that in the assay of the present invention, the target nucleic acid sequence is cleaved by the Ago enzyme of the present invention in the presence of primary guide ssDNAs to form a new 5' phosphorylated nucleic acid sequence (secondary guide ssDNAs); the secondary guide ssDNAs continue in the presence of PfAgo enzymes that the PfAgo enzyme is guided to cleave a fluorescent reporter nucleic acid complementary to the secondary guide ssDNAs. This specific cleavage of the target nucleic acid sequence (first cleavage) followed by a specific cleavage of the fluorescent reporter nucleic acid (second cleavage) is defined as a "secondary cleavage". In the present invention, both the first cleavage and the second cleavage are specific cleavages.

### Ago enzymes

A core component in the present invention and assay is a gene editing enzyme, such as an Ago enzyme.

In the present invention, the preferred Ago enzyme is the PfAgo enzyme, which is derived from the archaeon *Pyrococcus furiosus* and has a gene length of 2313 bp and an amino acid sequence consisting of 770 amino acids.

The cleavage characteristics of the PfAgo enzyme are: the enzyme can use the 5' phosphorylated oligonucleotide as a guide ssDNA to direct the precise cleavage of the target nucleic acid sequence by the enzyme; the cleavage site is located at the phosphodiester bond between the target nucleic acid (ssDNA) corresponding to nucleotides 10 and 11 of the guide ssDNA.

Typically, the preferred operating temperature for the PfAgo enzyme is 95 ± 2 degrees.

### Guide ssDNA

In the detection method of the present invention, a core component is a guide ssDNA, in particular 2 ssDNAs, and are adjacent to each other and have no spacer bases or spacer sequences between them.

In the present invention, the preferred guide ssDNAs are all oligonucleotides of length 10-60 nt, preferably 10-40 nt, more preferably 13-20 nt, its 5' first nucleotide is all thymine (T), which can be modified by phosphorylation.

### Reporter nucleic acid molecule

In the detection method of the present invention, a core component is a reporter nucleic acid carrying a reporter molecule.

In a preferred embodiment, the reporter nucleic acid molecule of the invention is a nucleic acid molecule carrying a fluorescent group and a quencher, respectively. For example, the fluorescent group (F) is labelled at the 5' end and the quencher (Q) at the 3' end.

In the present invention, the fluorescent reporter nucleic acid molecule is based on the position at which the secondary guide ssDNAs are generated; the target nucleic acid sequence is cleaved by the primary guide ssDNAs to form a new 5' phosphorylated nucleic acid sequence, called secondary guide ssDNAs, and the fluorescent reporter nucleic acid covers all positions of the secondary guide ssDNAs.

### Detection methods

Also provided in the present invention are methods for detecting nucleic acids based on the gene editing enzyme Ago, such as *Pyrococcus furiosus* Argonaute (PfAgo).

In the method of the present invention, based on the cleavage activity of the PfAgo enzyme, two guide ssDNAs can be designed based on a target nucleic acid molecule (e.g., single-stranded DNA, preferably amplified target nucleic acid molecule), which are adjacent to each other and have no spacer sequence, and the guide ssDNA targets the target nucleic acid molecule and mediates the cleavage of the target nucleic acid molecule by the PfAgo enzyme to form a new secondary guide ssDNA. The secondary guide ssDNA continues in the action of PfAgo enzyme, directing the PfAgo enzyme to cleave the fluorescent reporter nucleic acid complementary to the secondary guide ssDNAs, thus achieving detection of the target nucleic acid molecule. The method of the present invention can substantially improve the sensitivity and accuracy of target nucleic acid detection.

In the present invention, according to the design requirements of the guide ssDNAs, the PfAgo enzyme can be specially designed so that it can selectively cleave nucleic acid sequences in which there are differences in some of the loci, thereby achieving typing detection.

In the present invention, when used to distinguish different typing, the mutation sites corresponding to different typing are placed in the 10th and 11th positions of the guide ssDNAs when the guide ssDNAs are designed, and due to the selective specificity of the PfAgo enzyme, cleavage activity can be inhibited with two consecutive point mutations, resulting in the detection of different typing.

In the present invention, multiple target nucleic acid molecules and guide ssDNAs can be added simultaneously to the cleavage system of PfAgo enzyme, and the combination of reporter nucleic acids with different fluorescent groups can achieve multiple detection of the target nucleic acids.

The method of the present invention is well suited for the detection of trace nucleic acids. By combining reverse transcription LAMP and guide ssDNAs with specific sequences, the present invention allows the detection of target nucleic acid molecules at low concentrations of nucleic acid templates (220copies/mL).

In the present invention, the amplification primers used for the amplification reaction have a Tm value of typically about 65 ± 10 degrees and an amplified fragment size of about 90-200 bp. preferably, the amplification primers should be designed to avoid the region to be detected.

### Kit

The present invention also provides a detection kit for target nucleic acid molecules.

In a preferred embodiment, the kit of the present invention comprises: (a) an amplification reagent for amplifying a target nucleic acid molecule, the amplification reagent comprising: a primer pair for amplifying a target nucleic acid molecule, the primer pair being used to perform a specific amplification reaction based on the target nucleic acid molecule, thereby generating a specific nucleic acid amplification product;
(b) a cleavage reagent or a cleavage buffer comprising the cleavage reagent, wherein the cleavage reagent comprises: 2 guide ssDNA, a gene editing enzyme (Ago), and a first reporter nucleic acid, the fluorescent reporter nucleic acid bearing a fluorescent group and a quencher, and wherein the 2 guide ssDNA are adjacent to each other.

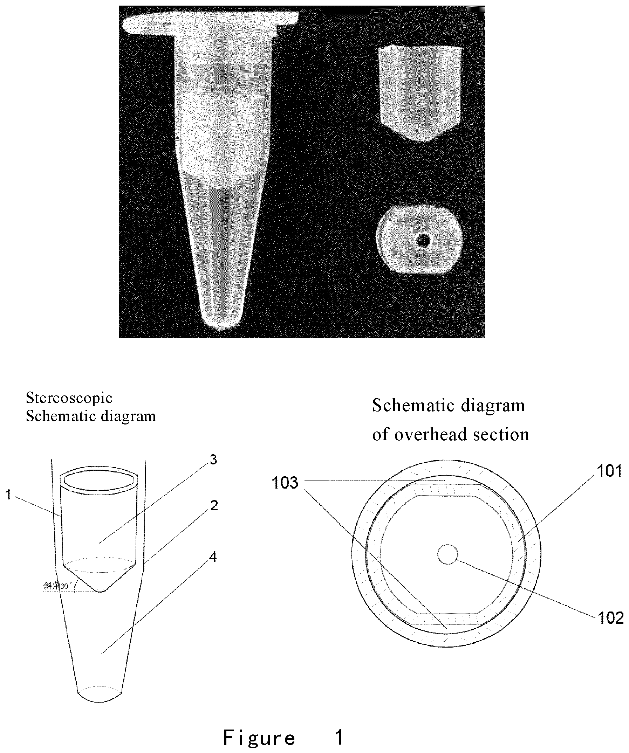

In a preferred embodiment, the kit of the present invention comprises:
(i) a first container and a guide ssDNA in the first container, the guide ssDNA is 2 and there is no spacer sequence between the 2 guide ssDNAs;
(ii) a second container and a gene editing enzyme (Ago) in the second container;
(iii) a third container and a first reporter nucleic acid in the third container, the first reporter nucleic acid bearing a fluorescent group and a quencher;
(iv) a fourth container and an amplification reagent for amplifying a target nucleic acid molecule in the fourth container; and
(v) optionally a fifth container and a buffer in the fifth container;
(vi) optionally a sixth container and a second reporter nucleic acid and a third reporter nucleic acid in the sixth container, the second reporter nucleic acid, the third reporter nucleic acid bearing a fluorescent group and a quencher;
(vi) optionally, a PCR tube and a liner pipe corresponding to the PCR (the liner pipe is shown in Figures 1-3);
wherein the target nucleic acid molecule comprises a single-stranded DNA.

### Applications

The present invention is particularly suitable for the detection of trace target nucleic acid molecules, as well as for multiple detection, and has a wide range of applications.

In the present invention, the target nucleic acid molecule can be DNA or RNA, and when the target nucleic acid molecule is RNA, it can be converted to cDNA by reverse transcription for further detection.

The present invention can achieve proactive management of disease monitoring, such as prediction and prevention, to achieve early detection and early treatment, or early prediction and early prevention. Since the detection sensitivity and accuracy of the present invention is very high, it is suitable for early diagnosis, symptomatic treatment, saving patients' treatment time and improving the success rate of treatment. The present invention reduces high medical cost waste, and strives for the golden time of treatment.

In environmental monitoring, the present invention can conveniently and rapidly identify nucleic acid molecules in environmental pollutants accurately and provide effective environmental detection data.

### The main advantages of the present invention include:

(1) Broad detection spectrum: the present invention has a broad nucleic acid detection spectrum, which enables efficient detection of viral, bacterial, and genetic disease genes;
(2) High sensitivity: the PfAgo specific nucleic acid cascade reaction system, which can be detected at low concentration of nucleic acid template (100copies/mL), has stability and reliability;
(3) Short time: detection of target genes can be achieved within 45 minutes.
(4) High reliability: the invention avoids false positives caused by non-specific pairing between ring primers in LAMP technique, and improves detection accuracy;
(5) POCT system: "One-tube" one-step detection of target nucleic acids such as novel coronaviruses, using a portable isothermal fluorescence system, in line with the POCT concept.
(6) Multiplex detection: The invention can also achieve multiplex detection in a single-tube reaction system, significantly reducing system complexity and cost of use.
(7) High fault tolerance for mutated bases: viruses with single- or double-base mutations in the amplified region can be detected.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Percentages and parts are percentages by weight and parts by weight, unless otherwise stated.

The experimental materials involved in the present invention are available from commercially available sources unless otherwise specified.

In the present invention, all sequence numbers refer to specific sequences, which do not carry any modifications.

### Example 1

### Preparation of detection reagent and detection method

In this embodiment, the isothermal rapid detection kit for SARS-CoV-2 virus nucleic acid of the present invention and its method of use are provided.

### 1.1 Detection reagents and kits

In this embodiment, taking the detection of ORFlb gene of SARS-CoV-2 virus as an example, the corresponding specific target nucleic acid sequences are:

5'-ATGCACTTTTCGCATATACAAAACGTAATGTCATCCCTACTATAACTCAA
ATGAATCTTAAGTATGCCATTAGTGCAAAGAATAGAGCTCGCACCGTAGCTGGT
GTCTCTATCTGTAGTACTATGACCAATAGACAGTTTCATCAAAAATTATTGAAAT
CAATAGCCGCCACTAGAGGAGCTACTGTAGTAATTGGAACAAGCAAATTCTATG
GTGGTTGGCACAACATGTTAAAAACTGTTTATAGTGATGTAGAAAACCCTCACC
TTATGGGTTGGGATTATCCTAAATGTGATAGAGCCATGCCTAACATGCTTAGAA
TTATGGCCTCACTTGTTCTTGCTCGCAAACATACAACGTGTTGTAGCTTGTCACA
CCGTTTCTATAGATTAGCTAATGAGTGTGCTCAAGTATTGAGTGAAATGGTCAT
GTGTGGCGGTTCACTATATGTTAAACCAGGTGGAACCTCATCAGGAGATGCCAC
AACTGCTTATGCTAATAGTGTTTTTAACATTTGTCAAGCTGTCACGGCCAATGTT
AATGCACTTTTATCTACTGATGGTAACAAAATTGCCGATAAGTATGTCCGCAAT
TTACAACACAGACTTTATGAGTGTCTCTATAGAAATAGAGATGTTGACACAGAC
TTTGTGAATGAGTTTTACGCATATTTGCGTAAACATTTCTCAATGATGATACTCT
CTGACGATGCTGTTGTGTGTTTCAATAGCACTTATGCATCTCAAGGTCTAGTGGC
TAGCATAAAGAACTTTAAGTCAGTTCTTTATTATCAAAACAATGTTTTTATGTCT
GAAGCAAAATGTTGGACTGAGACTGACCTTACTAAAGGACCTCATGAATTTTGC
TCTCAACATACAATGCTAGTTAAACAGGGTGATGATTATGTGTACCTTCCTTACC
CAGATCCATCAAGAATCCTAGGGGCCGGCTGTTTTGTAGATGATATCGTAAAAA
CAGATGGTACACTTATGATTGAACG-3', SEQ ID NO.: 65。

Based on the method of the present invention, the corresponding detection reagents include:
(1), amplification primers, the specific sequences are as follows:

| | | |
|---|---|---|
| F3 | 5'-TGTTCTTGCTCGCAAACA-3' | SEQ ID NO.13 |
| B3 | 5'-GTGTTGTAAATTGCGGACAT-3' | SEQ ID NO.14 |
| FIP | 5'-ACACATGACCATTTCACTCAATACTAGCTTGTCACACCGTTTC 3' | SEQ ID NO.15 |
| BIP | 5'-CATTTGTCAAGCTGTCACGGCGCAATTTTGTTACCATCAGTAG- 3' | SEQ ID NO.16 |
| LF2 | 5'-ACACTCATTAGCTAATC-3' | SEQ ID NO.17 |
| LB2 | 5'-CAATGTTAATGCACTTTT-3' | SEQ ID NO.18 |

(2), specific guide ssDNA (gDNA) with the following sequence:

| | | |
|---|---|---|
| 2019-nCoV ORF 1b-**gDNA 1** | 5'-**P**-TTGATGAGGTTCCACC-3' | SEQ ID NO.51 |
| 2019-nCoV ORF 1b-**gDNA 4** | 5'-**P**-TCAGTTGTGGCATCTC-3' | SEQ ID NO.52 |

(3), Fluorescent reporter nucleic acids corresponding to specific guide ssDNA (gDNA) with the following sequences:

| | | |
|---|---|---|
| 2019-nCoV ORF 1b-Reporter 1 | 5'-FAM-CAGTTGTGGCATCTCCTGATGAGG TTCCAC-BHQ1-3' | SEQ ID NO.60 |

(4), LAMP amplification buffer: Bst buffer (Tris-Hcl, EDTA, NaCl or KCl), MgSO₄, dNTP, DNase/RNase free H₂O.
(5), Ago digestion buffer: Bst buffer or Reaction buffer (Tris-Hcl, EDTA, NaCl or KCl), MnCl₂, DNase/RNase free H₂O.
(6), amplification enzymes: Bst polymerase, reverse transcriptase.
(7), Cleavage enzyme: PfAgo.
(7), PCR tubes and liner pipes corresponding to PCR tubes.

### 1.2 Detection method

The specific operation steps of the detection method of the isothermal rapid nucleic acid detection kit for SARS-CoV-2 virus of the present invention are as follows:
(1), the amplification primer, gDNA dry powder are dissolved with DNase/RNase free H₂O to make 100uM storage solution; the fluorescent reporter nucleic acid dry powder is dissolved with DNase/RNase free H₂O to make 10uM storage solution;
(2), preparation of 25 µl of amplification reaction premix with Bst enzyme and Ago digestion buffer amplification primers;
(3), add 15µl of the nucleic acid sample to be tested to the amplification reaction premix, and the amplification reaction system is 40µl;
(4), Preparation of 20 µl enzyme digestion reaction system with PfAgo, LAMP amplification buffer, gDNA, and fluorescent reporter nucleic acid;
(5), transfer the amplification system and the enzymatic digestion system into the PCR tube reaction chamber and the liner pipe reaction chamber, respectively, because of the surface tension of the liquid, the enzymatic digestion system in the liner pipe reaction chamber will not fall into the PCR tube reaction chamber, and put into the fluorescent quantitative PCR instrument for reaction (amplification at 65 °C for 30min, transient centrifugation, and then enzymatic digestion at 95 °C for 30min, detecting the signal once every minute).

### Example 2

Preparation of detection reagents and detection method (without using liner pipes)

In this Example, the isothermal rapid detection kit for SARS-CoV-2 virus nucleic acid of the present invention and its method of use are provided.

### 1.1 Detection reagents and kits

In this Example, take the detection of ORFlb gene of SARS-CoV-2 virus as an example and the corresponding specific target nucleic acid sequence is referred to Example 1.

Based on the method of the present invention, the corresponding detection reagents include:
(1), amplification primers with the following sequences:

| | | |
|---|---|---|
| F3 | 5'-TGTTCTTGCTCGCAAACA-3' | SEQ ID NO. 13 |
| B3 | 5'-GTGTTGTAAATTGCGGACAT-3' | SEQ ID NO. 14 |
| FIP | 5'-ACACATGACCATTTCACTCAATACTAGCTTGTCACACCGTTT C3' | SEQ ID NO.15 |
| BIP | 5'-CATTTGTCAAGCTGTCACGGCGCAATTTTGTTACCATCAGTA G-3' | SEQ ID NO. 16 |
| LF2 | 5'-ACACTCATTAGCTAATC-3' | SEQ ID NO. 17 |
| LB2 | 5'-CAATGTTAATGCACTTTT-3' | SEQ ID NO.18 |

(2), specific guide ssDNA (gDNA) with the following sequence:

| | | |
|---|---|---|
| 2019-nCoV ORF 1b-**gDNA 1** | 5'-**P**-TTGATGAGGTTCCACC-3' | SEQ ID NO.51 |
| 2019-nCoV ORF 1b-**gDNA 4** | 5'-**P**-TCAGTTGTGGCATCTC-3' | SEQ ID NO.52 |

(3), Fluorescent reporter nucleic acids corresponding to specific guide ssDNA (gDNA) with the following sequences:

| | | |
|---|---|---|
| 2019-nCoV ORF 1b-Reporter 1 | 5'-FAM-CAGTTGTGGCATCTCCTGATG AGGTTCCAC-BHQ1-3' | SEQ ID NO.60 |

(4), LAMP amplification buffer: Bst buffer (Tris-Hcl, EDTA, NaCl or KCl), MgSO₄, dNTP, DNase/RNase free H₂O.
(5), Ago digestion buffer: Bst buffer or Reaction buffer (Tris-Hcl, EDTA, NaCl or KCl), MnCl₂, DNase/RNase free H₂O.
(6), amplification enzymes: Bst polymerase, reverse transcriptase.
(7), Cleavage enzyme: PfAgo.
(8), PCR tubes.

### 1.2 Detection method

The specific operation steps of the detection method of the isothermal rapid nucleic acid detection kit for SARS-CoV-2 virus of the present invention are as follows:
(1), the amplification primer, gDNA dry powder are dissolved with DNase/RNase free H₂O to make 100uM storage solution; the fluorescent reporter nucleic acid dry powder is dissolved with DNase/RNase free H₂O to make 10uM storage solution;
(2), Bst enzyme, Ago digestion buffer and amplification primers were used to prepare 25 µl of amplification reaction premix;
(3), add 15 µl of nucleic acid samples to be tested to the amplification reaction premix, and the amplification reaction system is 40 µl;
(4), The amplification system was placed in a fluorescent quantitative PCR instrument for the reaction and amplified at 65°C for 30 min;
(5), Prepare 20µl enzyme digestion reaction system with PfAgo, LAMP amplification buffer, gDNA, fluorescent reporter nucleic acid, add the enzyme digestion reaction system to the reaction completed amplification system, and then put it into the fluorescent quantitative PCR instrument, digestion at 95°C for 30min, and the signal is detected once every minute.

### Example 3

### Detection for different concentrations of standards to be tested

The standards to be tested (SEQ ID NO.: 65) were diluted to 18000copies/mL, 6000copies/mL, 2000copies/mL, 670copies/mL, 220copies/mL, 70copies/mL, respectively, according to the principle of 3-fold multiple proportion dilution. 140µl of standard dilutions were aspirated for nucleic acid extraction (QIAamp Viral RNA Mini Kit ). Add 15µl of nucleic acid extraction sample, negative control (H2O) and positive control (target fragment plasmid) to the amplification system in Example 2, make 3 groups for each concentration gradient, make 7 replicates for each group, and calculate the detection rate for each group, and the experiment was performed according to the steps of Example 3.

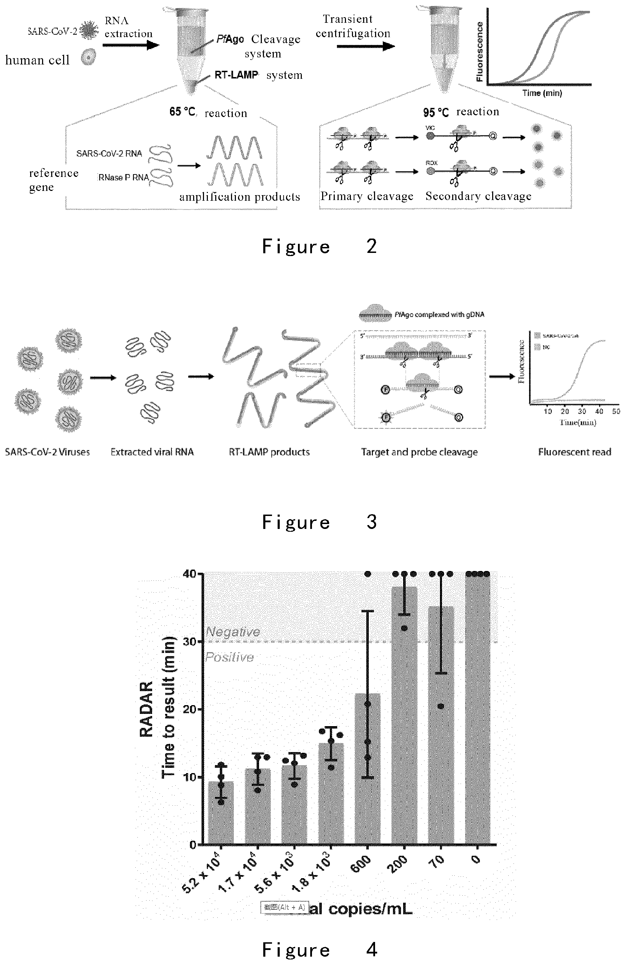

The results are shown in Figure 4. 18000copies/mL, 6000copies/mL, 2000copies/mL and 670copies/mL are stably detected, and the negative control and positive control are established.

The results show that the lowest detection limit of SARS-CoV-2 virus of the present invention can reach 670copies/mL.

### Example 4

### Dual detection of clinical samples

Six clinical samples confirmed by 2 fluorescent RT-PCR methods were used.

It should be noted that the RNase P reference gene is part of the human genome used as an internal quality control.

Configuration of the dual reaction system: RNase P reference gene amplification primers (primer pair 7) were added to the amplification system in Example 2, RNase P gDNA (SEQ ID NO. 57 and SEQ ID NO. 58) and RNase P fluorescent reporter nucleic acid (SEQ ID NO. 64) were added to the digestion system, and the reaction conditions were referred to Example 2.

| | | | |
|---|---|---|---|
| Prime r pair 7 | F3 | TTGATGAGCTGGAGCCA | SEQ ID NO.37 |
| | B3 | CACCCTCAATGCAGAGTC | SEQ ID NO.38 |
| | FIP | GTGTGACCCTGAAGACTCGGTTTTAGCCACTGACTCGG ATC | SEQ ID NO.39 |
| | BIP | CCTCCGTGATATGGCTCTTCGTTTTTTTCTTACATGGCT CTGGTC | SEQ ID NO.40 |
| | LF1 | ATGTGGATGGCTGAGTTGTT | SEQ ID NO.41 |
| | LB1 | CATGCTGAGTACTGGACCTC | SEQ ID NO.42 |

| | | |
|---|---|---|
| RNase P**-gDNA1** | 5'-**P**-TACTCAGCATGCGAAG-3' | SEQ ID NO.57 |
| RNase P**-gDNA2** | 5'-**P**-TGCCATATCACGGAGG-3' | SEQ ID NO.58 |
| RNase P-Reporter2 | 5'-VIC-CTCAGCATGCGAAGAGCCATATC ACGGAGG-BHQ1-3' | SEQ ID NO.64 |

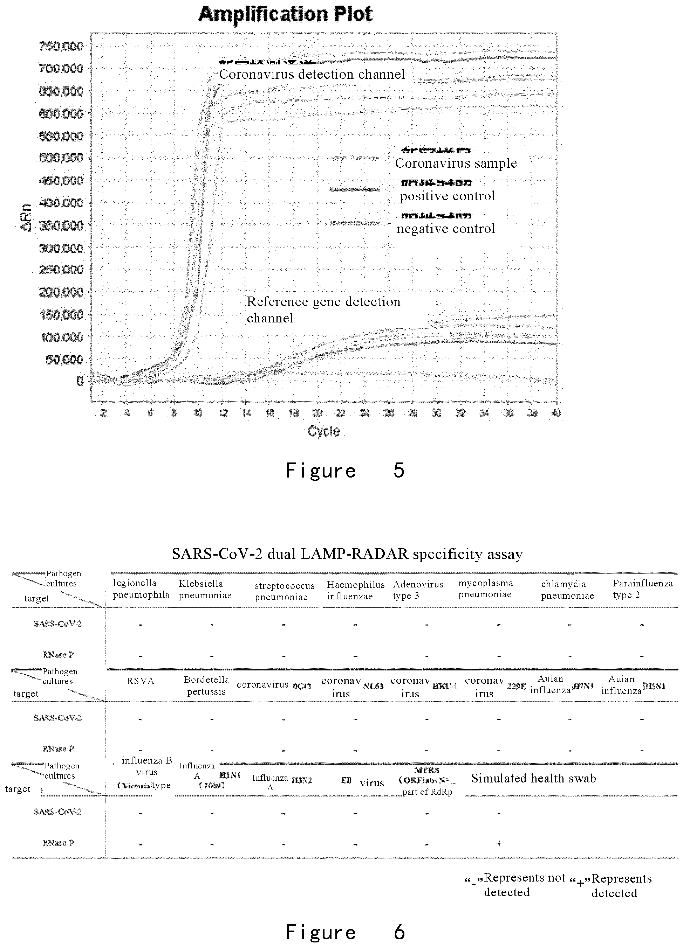

The results are shown in Figure 5. When the target nucleic acids detected are ORFlb gene and RNase P gene, FAM and VIC generate fluorescent signals simultaneously. The results show that the method of the present invention can be used for single-tube dual detection.

### Example 5

### Specificity detection

The full-length nucleic acid samples of 21 common respiratory pathogens, the full-length nucleic acid samples of human genome and the full-length nucleic acid samples of SARS-CoV-2 were used as the nucleic acid samples to be tested, and the system configuration and amplification and enzymatic digestion reactions were performed with reference to Example 4.

The results are shown in Figure 6. 21 common respiratory pathogens full-length nucleic acid samples do not produce signals, human genome full-length nucleic acid samples produce VIC signals and SARS-CoV-2 full-length nucleic acid samples produce FAM signals. The results indicate that the method of the present invention has good specificity.

### Example 6

### Typing detection of mutant strains

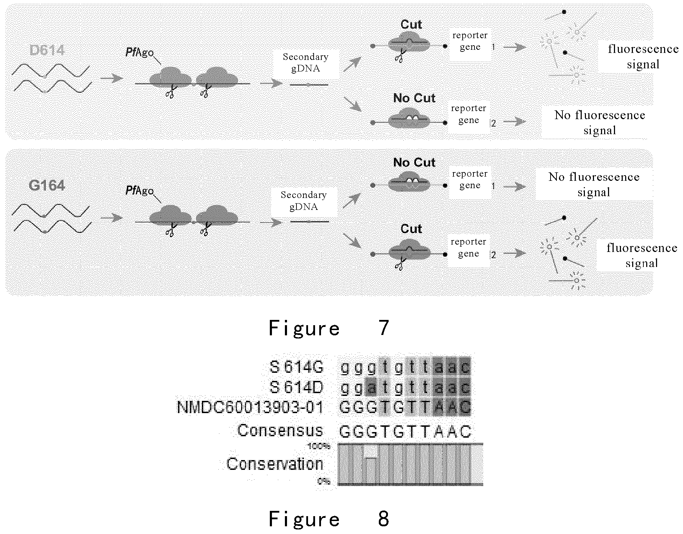

The D614G mutation appeared in the Beijing new coronavirus case with an almost 10-fold increase in infectivity, as shown in Figure 8, D614A>G i.e. A to G.

This mutant strain has strong pathogenicity and the possibility of outbreak is not excluded, for which the present invention is designed for typing detection. Because it is difficult to do single base typing by RT-PCR, ddPCR and sequencing methods are time-consuming, expensive, etc. The present invention can be flexibly designed to differentiate detection of known mutations, the specific principle is shown in Figure 7.

Differential detection of SARS-CoV-2 virus D614G mutation from SARS-CoV-2 virus nucleic acid sequence by combining the technique of the present invention (LAMP-RADAR), the specific design is as follows:
On the basis of the original design (double-guide DNA), two additional Reporter were designed. one set of gDNA retained the design of the original SARS-CoV-2 detection site, and the other two Reporter introduced one or two consecutive mismatches relative to position 10 or 11 of the secondary gDNA, so as to guarantee that the secondary gDNA has at least one mismatch relative to the newly designed Reporter. The detailed design of Reporter is as follows (Reporter with FAM probe and VIC probe as an example): design D614 (wild type) secondary gDNA with only one mismatch to the FAM probe and two consecutive mismatches with VIC probe, so that PfAgo cuts only the FAM probe and not the VIC probe under D614 (wild type) secondary gDNA mediation; while the G614 (mutant) secondary gDNA has two consecutive mismatches with the FAM probe and only one mismatch with the VIC probe, thus under the G614 (mutant) secondary gDNA mediation, PfAgo only cuts the VIC probe and not the FAM probe. Based on the above design, the D614G typing detection was achieved by determining the D614 type and G614 type based on the fluorescence signal generation of the two Reporter.

The corresponding sequences of synthetic reporter were designed as follows:
D614-FAM-11G: 5'FAM-CTGTGCAGTTAACGTCCTGATAAAGAACAG (SEQ ID NO.66)-BHQ1 3'
D614-FAM-11C: 5'FAM-CTGTGCAGTTAACCTCCTGATAAAGAACAG(SEQ ID NO.67)-BHQ1 3'
D614-FAM-11T: 5'FAM-CTGTGCAGTTAACTTCCTGATAAAGAACAG(SEQ ID NO.68)-BHQ1 3'
G614-VIC-11T: 5'VIC-CTGTGCAGTTAACTCCCTGATAAAGAACAG(SEQ ID NO.69)-BHQ1 3'
G614-VIC-11G: 5'VIC-CTGTGCAGTTAACGCCCTGATAAAGAACAG(SEQ ID NO.70)-BHQ1 3'
G614-VIC-11C: 5'VIC-CTGTGCAGTTAACCCCCTGATAAAGAACAG(SEQ ID NO.71)-BHQ1 3'

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

## Claims

1. A method for detecting a target nucleic acid molecule, comprising the steps of:
(a) providing a sample to be tested comprising a target nucleic acid molecule, the target nucleic acid molecule comprises a single stranded DNA;
(b) mixing the sample to be tested with a cleavage reagent or a cleavage buffer containing the cleavage reagent, thereby forming a detection system, wherein the cleavage reagent comprises: 2 guide ssDNA, a gene editing enzyme (Ago), and a first reporter nucleic acid molecule, the first reporter nucleic acid molecule bearing a fluorescent group and a quencher, and wherein the 2 guide ssDNA are adjacent to each other; and
(c) performing a fluorescence detection on the detection system, thereby obtaining a fluorescence signal value, wherein the detection of a fluorescence signal value in the detection system indicates the presence of a target nucleic acid molecule in the sample, and the absence of a fluorescence signal value in the detection system indicates the absence of a target nucleic acid molecule in the sample.

2. The method of claim 1, wherein the sample to be tested comprises an unamplified sample as well as an amplified (or nucleic acid amplified) sample.

3. The method of claim 1, wherein the first nucleotide at the 5' end of the respective guide ssDNA is T.

4. The method of claim 1, wherein the lengths of the guide ssDNA are each independently 10-60 nt, preferably 10-40 nt, more preferably, 13-20 nt.

5. The method of claim 1, wherein the guide ssDNA is a phosphorylated single stranded DNA molecule.

6. The method of claim 1, wherein the gene editing enzyme Ago is selected from the group consisting of: *PfAgo (Pyrococcus furiosus Ago), MfAgo (Methanocaldococcus fervens Ago), TcAgo (Thermogladius calderae Ago), TfAgo ( Thermus filiformis Ago), AaAgo (Aquifex aeolicus Ago), TpAgo (Thermus parvatiensis Ago),* and a combination thereof.

7. The method of claim 1, wherein the first reporter nucleic acid molecule has a length of 9-100nt, preferably 10-60nt, more preferably 15-40nt.

8. The method of claim 1, wherein the target nucleic acid molecule is selected from the group consisting of: a nucleic acid molecule of a pathogenic microorganism, a nucleic acid molecule with a genetic mutation, and a specific target nucleic acid molecule.

9. A kit for the detection of a target nucleic acid molecule, the kit comprising.
(a) an amplification reagent for amplifying a target nucleic acid molecule, the amplification reagent comprising: a primer pair for amplifying a target nucleic acid molecule, the primer pair being used to carry out a specific amplification reaction based on the target nucleic acid molecule, thereby producing a specific nucleic acid amplification product, thereby producing a specific nucleic acid amplification product.
(b) a cleavage reagent or a cleavage buffer comprising the cleavage reagent, wherein the cleavage reagent comprises: 2 guide ssDNA, a gene editing enzyme (Ago), and a first reporter nucleic acid, the first reporter nucleic acid bearing a fluorescent group and a quencher, and wherein the 2 guide ssDNA are adjacent to each other.

10. A kit for detecting a target nucleic acid molecule, the kit comprising:
(i) a first container and a guide ssDNA in the first container, the guide ssDNA being 2 and the 2 guide ssDNA being adjacent to each other;
(ii) a second container and a gene editing enzyme (Ago) in the second container;
(iii) a third container and a first reporter nucleic acid in the third container, the first reporter nucleic acid bearing a fluorescent group and a quencher;
(iv) a fourth container and an amplification reagent for amplifying a target nucleic acid molecule in the fourth container; and
(v) optionally a fifth container and a buffer in the fifth container;
(vi) optionally a PCR tube and a liner pipe corresponding to the PCR; wherein the target nucleic acid molecule comprises a single stranded DNA.
